Europäisches Patentamt

European Patent Office

Office européen des brevets

(19)

(11) **EP 1 285 054 B1**

(12) **EUROPEAN PATENT SPECIFICATION**

(45) Date of publication and mention
of the grant of the patent:
**04.08.2004 Bulletin 2004/32**

(51) Int Cl.[7]: **C11D 3/384**, D06M 16/00,
D06L 1/00, C07K 19/00

(21) Application number: **01936318.3**

(22) Date of filing: **03.05.2001**

(86) International application number:
**PCT/EP2001/005005**

(87) International publication number:
**WO 2001/092452 (06.12.2001 Gazette 2001/49)**

(54) **PROCESS FOR BINDING AN ANTIGEN TO A MOLECULE HAVING A HIGH BINDING AFFINITY TO SAID ANTIGEN**

VERFAHREN ZUR BINDUNG EINES ANTIGENS AN EIN MOLEKÜL MIT HOHER
BINDUNGSAFFINITÄT ZUM ANTIGEN

PROCEDE SERVANT A FIXER UN ANTIGENE A UNE MOLECULE PRESENTANT UNE AFFINITE
ELEVEE DE FIXATION AUDIT ANTIGENE

(84) Designated Contracting States:
**AT BE CH CY DE DK ES FI FR GB GR IE IT LI LU
MC NL PT SE TR**

(30) Priority: **31.05.2000 EP 00304630**

(43) Date of publication of application:
**26.02.2003 Bulletin 2003/09**

(73) Proprietors:
  • **UNILEVER N.V.**
    **3013 AL Rotterdam (NL)**
    Designated Contracting States:
    **AT BE CH DE DK ES FI FR GR IT LI LU MC NL PT
    SE TR**
  • **UNILEVER PLC**
    **London EC4P 4BQ (GB)**
    Designated Contracting States:
    **CY GB IE**

(72) Inventors:
  • **HEMMINGTON, Sandra**
    **Bedford MK40 3SZ (GB)**
  • **LITTLE, Julie**
    **Sharnbrook, Bedfordshire MK44 1LQ (GB)**
  • **PARRY, Neil James**
    **Sharnbrook, Bedfordshire MK44 1LQ (GB)**

(74) Representative: **Kan, Jacob Hendrik**
    **Unilever N.V.**
    **Patent Department,**
    **Olivier van Noortlaan 120**
    **3133 AT Vlaardingen (NL)**

(56) References cited:
  **WO-A-01/46514**          **WO-A-98/00500**
  **WO-A-98/23716**          **WO-A-98/56885**

**Description**

## TECHNICAL FIELD

The present invention generally relates to the field of biochemistry. More in particular, it relates to a process for binding an antigen to a molecule having a high binding affinity to said antigen, such as an antibody. It especially relates to a process for binding an antigen to a molecule having a high binding affinity, whereby the binding is carried out in a medium having a water content of less than 25% by weight of the total composition.

## BACKGROUND AND PRIOR ART

Antigens are molecules or molecular structures which can be recognised by molecules which bind to them and have a high affinity for them. Most biochemical processes, including the recognition of antigens by antibodies, occur in nature in essentially aqueous media. Also in industry, the application of antibodies to recognise their respective targets is traditionally performed in the presence of a liquid aqueous environment. For instance, WO-A-98/56885 (Unilever) discloses in the examples that stains on fabric may be bleached in an aqueous environment by means of glucose oxidase enzyme, coupled to an antibody raised against compounds present in the stains.

WO-A-98/00500 (Unilever) discloses a laundry composition comprising a benefit agent attached to a peptide or protein deposition aid, which has a high affinity for fabric. The composition is claimed to effectively deposit the benefit agent onto the fabric during the wash cycle.

According to DE-A-196 21 224 (Henkel), the transfer of textile dyes from one garment to another during a washing or rinsing process may be inhibited by adding antibodies against the textile dye to the wash or rinse liquid.

WO-A-98/07820 (P&G) discloses amongst others rinse treatment compositions containing antibodies directed at cellulase and standard softener actives (such as DEQA).

The earlier, not prepublished WO-A-01/46514 (Unilever) discloses a method of delivering a benefit agent to a selected area of a fabric for exerting a predetermined activity, which comprises pre-treating said area with a multi-specific binding molecule, said binding molecule having a high binding affinity to said area through one specificity and is capable of binding to said benefit agent through another specificity, followed by contacting said pre-treated area with said benefit agent to exert said pre-determined activity to said area. Also described is a device for use in the above-mentioned method, in the form of a dispenser capable of depositing a multi-specific binding molecule to a selected area of a fabric, said binding molecule having a high binding affinity through one specificity to said area and is capable of binding to a benefit agent through another specificity, through a semi-solid wax or soap-stick, roll-on pen, spray, aerosol, impregnated brush, gel, or foam.

Therefore, up to now, the binding reaction between an antigen and the molecule having a high binding affinity to said antigen has always been carried out in aqueous or essentially aqueous media. We have now surprisingly found that the binding of an antigen to a molecule having a high binding affinity to said antigen is inhibited to a much lesser degree than expected at low water concentrations, e.g. in a medium having a water content of less than 25% by weight of the total composition. This makes it possible to formulate products which were until now deemed to be ineffective and unsuccessful.

## DEFINITION OF THE INVENTION

According to the invention, there is provided a process for binding an antigen to a molecule having a high binding affinity to said antigen, characterised in that the binding is carried out in a medium having a water content of less than 25% by weight of the total composition.

## DETAILED DESCRIPTION OF THE INVENTION

### 1. The antigen

The invention relates to a process whereby an antigen is bound to a molecule having a high binding affinity to said antigen, whereby the binding is carried out in a medium having a water content of less than 25% by weight of the total composition. Preferably the water content is less than 15%, more preferably less that 10% by weight of the total composition. The lower limit of the water content is for practical reasons more than about 1%, usually 5% or 10% by weight of the total composition. The delivery of antibodies to antigens in a low water environment can be carried out in a liquid environment but lacking water (e.g. glycerol substituted) or in a totally dried format (e.g. either powder or particulate) or in laundry dry cleaning conditions (solvent or liquid CO2 washing).

The antigen can be any molecular structure that is capable of being recognised by a corresponding antibody molecule, protein fragment or derivative thereof.

### 2.1 The molecule having a high binding affinity.

In the process according to the invention, the antigen is bound by means of a reagent having a high binding affinity for the antigen. A specific example of such a reagent is for instance an antibody.

Generally speaking, the degree of binding of a molecule A to another molecule B can be generally expressed by the chemical equilibrium constant $K_d$ resulting from the following reaction:

$$[A]+[B] \Leftrightarrow [AB]$$

The chemical equilibrium constant $K_d$ is then given by:

$$K_d = \frac{[A]x[B]}{[AB]}$$

Whether the binding of a molecule to the fabric is specific or not can be judged from the difference between the binding ($K_d$ value) of the molecule to one type of fabric, versus the binding to another type of fabric material. For applications in laundry, said material will be a fabric such as cotton or polyester. However, it will usually be more convenient to measure $K_d$ values and differences in $K_d$ values on other materials such as a polystyrene microtitre plate or a specialised surface in an analytical biosensor. The difference between the two binding constants should be minimally 10, preferably more than 100, and more preferably, more that 1000. Typically, the reagent should bind to the fabric, with a $K_d$ lower than $10^{-4}$ M, preferably lower than $10^{-6}$ M and could be $10^{-10}$ M or even less. Higher binding affinities ($K_d$ of less than $10^{-5}$ M) and/or a larger difference between the one type of fabric and another type of fabric (or background) binding would increase the deposition of the benefit agent. Also, the weight efficiency of the reagent in the total rinse composition would be increased and smaller amounts of the reagent would be required.

Several classes of reagent or molecules can be envisaged which deliver the capability of specific binding to fabrics, to which one would like to deliver the benefit agent. In the following we will give a number of examples of such molecules having such capabilities, without pretending to be exhaustive.

2.1.1. Antibodies.

Antibodies are specific binding proteins. Their function in nature is to protect against disease by recognising (and binding) foreign bodies, such as viruses or Bacteria, but not self-cells. Furthermore, methods are well-known in the art to generate antibodies that are specific for almost any protein, organic molecule, or cell surface, that is likely to be encountered. This binding specificity has been exploited in the Biotechnology industry, principally for medical diagnostics. For example, many home-based pregnancy test kits comprise an antibody that specifically binds to the pregnancy marker hormone, human chorionic gonadotrophin (hCG), but not to other hormones present in urine.

More recently, the use of antibodies in laundry products has been described (Henkel, Procter and Gamble, Unilever). In particular, Unilever has described the use of stain-specific antibodies to target bleaching enzymes exclusively to stains but not to dyes - thus achieving efficient stain removal without damaging surrounding fabric.

Antibodies are well known examples of molecules which are capable of binding specifically to compounds against which they were raised. Antibodies can be derived from several sources. From mice, monoclonal antibodies can be obtained which possess very high binding affinities. From such antibodies, Fab, Fv or scFv fragments, can be prepared which have retained their binding properties. Such antibodies or fragments can be produced through recombinant DNA technology by microbial fermentation. Well known production hosts for antibodies and their fragments are yeast, moulds or bacteria.

A class of antibodies of particular interest is formed by the Heavy Chain antibodies as found in Camelidae, like the camel or the 11ama. The binding domains of these antibodies consist of a single polypeptide fragment, namely the variable region of the heavy chain polypeptide (HC-V). In contrast, in the classic antibodies (murine, human, etc.), the binding domain consists of two polypeptide chains (the variable regions of the heavy chain ($V_h$) and the light chain ($V_1$)). Procedures to obtain heavy chain immunoglobulins from Camelidae, or (functionalized) fragments thereof, have been described in WO-A-94/04678 (Casterman and Hamers) and WO-A-94/25591 (Unilever and Free University of Brussels).

Alternatively, binding domains can be obtained from the $V_h$ fragments of classical antibodies by a procedure termed "camelization". Hereby the classical $V_h$ fragment is transformed, by substitution of a number of amino acids, into a HC-V-like fragment, whereby its binding properties are retained. This procedure has been described by Riechmann et al. in a number of publications (J. Mol. Biol. (1996) **259**, 957-969; Protein. Eng. (1996) **9**, 531-537, Bio/Technology (1995) 13, 475-479). Also HC-V fragments can be produced through recombinant DNA technology in a number of microbial hosts (bacterial, yeast, mould), as described in WO-A-94/29457 (Unilever).

Methods for producing fusion proteins that comprise an enzyme and an antibody or that comprise an enzyme and an antibody fragment are already known in the art. One approach is described by Neuberger and Rabbits (EP-A-194 276). A method for producing a fusion protein comprising an enzyme and an antibody fragment that was derived from an antibody originating in *Camelidae* is described in WO-A-94/25591. A method for producing bispecific antibody fragments is described by Holliger et al. (1993) PNAS 90, 6444-6448.

A particularly attractive feature of antibody binding behaviour is their reported ability to bind to a "family" of structurally related molecules. For example, in Gani et al. (J. Steroid Biochem. Molec. Biol. **48**, 277-282) an antibody is described that was raised against progesterone but also binds to the structurally-related steroids, pregnanedione, pregnanolone and 6-hydroxy-progesterone. Therefore, using the same approach, antibodies could be isolated that bind to a whole "family" of stain chromophores (such as the polyphenols, porphyrins, or caretenoids as described below). A broad action antibody such as this could be used to treat several different stains when coupled to a bleaching enzyme.

### 2.1.2. Peptides.

Peptides usually have lower binding affinities to the substances of interest than antibodies. Nevertheless, the binding properties of carefully selected or designed peptides can be sufficient to deliver the desired selectivity in an oxidation process. A peptide which is capable of binding selectively to a fabric to which one would like to deliver a benefit agent, can for instance be obtained from a protein which is known to bind to that specific fabric. An example of such a peptide would be a binding region extracted from an antibody raised against that fabric. A suitable peptide could be analogous to the active center of a protein analogous to a non-catalytic binding domain of a protein, e.g. a receptor.

Alternatively, peptides that bind to such substances can be obtained by the use of peptide combinatorial libraries. Such a library may contain up to $10^{10}$ peptides, from which the peptide with the desired binding properties can be isolated. (R.A. Houghten, Trends in Genetics, Vol 9, no &, 235-239). Several embodiments have been described for this procedure (J. Scott et al., Science (1990) **249**, 386-390; Fodor et al., Science (1991) 251, 767-773; K. Lam et al., Nature (1991) **354**, 82-84; R.A. Houghten et al., Nature (1991) **354**, 84-86).

Suitable peptides can be produced by organic synthesis, using for example the Merrifield procedure (Merrifield (1963) J.Am.Chem.Soc. **85**, 2149-2154). Alternatively, the peptides can be produced by recombinant DNA technology in microbial hosts (yeast, moulds, bacteria)(K.N. Faber et al. (1996) Appl. Microbiol. Biotechnol. **45**, 72-79).

### 2.1.3. Pepidomimics.

In order to improve the stability and/or binding properties of a peptide, the molecule can be modified by the incorporation of non-natural amino acids and/or non-natural chemical linkages between the amino acids. Such molecules are called peptidomimics (H.U. Saragovi et al. (1991) Bio/Technology **10**, 773-778; S. Chen et al. (1992) Proc.Natl.Acad. Sci. USA **89**, 5872-5876). The production of such compounds is restricted to chemical synthesis.

### 2.1.4. Other organic molecules.

It can be readily envisaged that other molecular structures, which need not be related to proteins, peptides or derivatives thereof, can be found which bind selectively to fabrics to which one would like to deliver a benefit agent. For example, certain polymeric RNA molecules which have been shown to bind small synthetic dye molecules (A. Ellington et al. (1990) Nature 346, 818-822). Such binding compounds can be obtained by the combinatorial approach, as described for peptides (L.B. McGown et al. (1995), Analytical Chemistry, 663A-668A).

This approach can also be applied for purely organic compounds which are not polymeric. Combinatorial procedures for synthesis and selection for the desired binding properties have been described for such compounds (Weber et al. (1995) Angew.Chem. Int.Ed. Engl. **34**, 2280-2282; G. Lowe (1995), Chemical Society Reviews **24**, 309-317; L.A. Thompson et al. (1996) Chem. Rev. **96**, 550-600). Once suitable binding compounds have been identified, they can be produced on a larger scale by means of organic synthesis.

The molecule has a high binding affinity for the antigen. If the molecule is a protein or a peptide, it may be that one part of its polypeptide chain is responsible for the binding affinity to the fabric, and part of the reagent comprises an enzyme part capable of providing some kind of benefit. In the first situation, the enzyme may be a fusion protein comprising two domains, which may be coupled by means of a linker. Alternatively, the molecule having the high binding affinity may be covalently coupled to a benefit agent by means of a bivalent coupling agent such as glutardialdehyde. A full review of chemistries appropriate for coupling two biomolecules is provided in "Bioconjugate techniques" by Greg T. Hermanson, Academic Press Inc (1986). Alternatively, if the molecule having the high binding affinity is a peptide or a protein, it may also be coupled to the enzyme by constructing a fusion protein. In such a construct there would typically be a peptide linker between the binding reagent and the enzyme. An example of a fusion of an enzyme and a binding reagent is described in Ducancel et al. Bio/technology 11, 601-605.

A further embodiment would be for the molecule with the high binding affinity to be a bispecific molecule. Such a molecule could fulfil the requirement of accumulating a benefit agent on the fabric either by supplying said molecule together with the benefit agent as a pre-formed non-covalent complex or by supplying the two separately and allowing them to self-assemble either in the wash- or rinse liquor or on the fabric.

In a preferred embodiment, the process of the invention is carried out using micro-particles sensitised with an antigen or antibody / antigen combinations configured such that the micro-particles are loaded with a benefit agent and the antibody has a high affinity or specificity for a substance (or "marker molecule") typically found on some regions of fabrics but not on others. Examples of such marker molecules include bleach-damaged dyes and microbes known to be associated with malodour. The antibody targets the benefit agent to its intended site of action and binds it there. For example, Microbe-specific antibodies may target fragrance-containing particles to the regions of malodour. Thus, a more efficient use of expensive ingredients is achieved. Alternatively, antibodies specific for bleach-damaged dyes can target dyed particles to faded regions, thus replenishing the colour lost in the main wash cycle.

Previously, such micro-particles have been sensitised with antibody and used to generate a coloured end-point in medical diagnostic devices, when they are applied manually onto a test strip. According to the present invention, analogous particles are being specifically bound to some cotton swatches but not others, depending on which marker molecules are present on the swatches. The binding of particles is being driven not by manual application but by agitating a bulk liquid phase (e.g. a rinse liquor) containing said particles and swatches. The agitation increases the number of collisions between fabric and particles and thus increases specific binding: particles sensitised with specific antibody result in productive collisions and binding is permanent; particles sensitised with non-specific antibody result in non-productive collisions and do not bind permanently. Such an agitation could be readily achieved during the rinse cycle in an automatic washing machine.

Another advantage of the present invention is that it is possible to target some benefit molecules to particular regions of fabric during the rinse. For example, dyes can be targeted to colour-bleached regions to replenish dye lost in the main wash or fragrance can be targeted to regions where it is most needed (i.e. those regions where microbes associated with malodour are present - such as the "underarm" regions). However, methods for targeting small molecules (such as a dye or a fragrance) to particular regions of fabric have not previously been described. The inventors have approached this problem by loading small molecules (such as a dye) onto a micro-particle and then sensitising the particles with an antibody. The advantage of this is that a single antibody binding event can deposit many dye molecules onto the target-region of fabric. Whereas antibody-sensitised particles have been described previously (as component parts of medical diagnostic devices) they are used in a fundamentally different way: in the medical device, the particles are manually applied to the target surface (typically nitro-cellulose paper) and then eluted with a solution. If specific antibody is present, the particles remain stuck on. Otherwise they do not. In contrast, antibody-sensitised particles have not previously been used to target a small chemical compound (such as a dye or a fragrance) from a bulk liquid phase to a particular target site on a surface. Furthermore, if an analogous interaction between the particles and the target surface (i.e. if the swatches are manually placed in the bulk liquid and left static) little or no binding is observed. However, the inventors have been able to achieve surprisingly specific binding to cotton swatches by agitating a bulk liquid phase (of rinse liquor or tap water) containing said particles and swatches.

For laundry detergent applications, several classes of natural or man-made fabrics can be envisaged, in particular cotton. Such macromolecular compounds have the advantage that they can have a more immunogenic nature, i.e. that it is easier to raise antibodies against them. Furthermore, they are more accessible at the surface of the fabric than for instance coloured substances in stains, which generally have a low molecular weight.

An important embodiment of the invention is to use a binding reagent (as described above) that binds to several different types of fabrics. This would have the advantage of enabling a single benefit agent to be deposited to several different types of fabric.

3. Applications

The invention can be carried out in various systems. First, the antibodies can be delivered in a spray system. Typical spray systems are:

**3.1. Pump systems**

These use mechanical force usually applied by squeezing a handle (e.g. sprays used to water plants)

**3.2. Antiperspirant aerosols**

These are suspensions of the solid active compound (an aluminium salt) in silicone oils.
10% Aluminium salt
13 % Cyclomethicone
1% Bentone 38 (Quaternium 18 Hectorite)
1% Fragrance
75% Propellant (called CAP 40 which is a mix of 22% propane, 24% iso-butan and 54% n-butane)
77.1g of propellant into a 150ml aerosol can.

**3.3. Deodorant aerosol**

These can vary quite a lot from country to country, but are roughly:
7.5g ethanol
1g isopropyl mystirate (helps reduce stinging on application) 1.5 g Fragrance
35g CAP 40 propellant,
In a 150 ml can.

**3.4. Hairspray**

They use either dimethyl ether or CAP 40 as the propellant. Hairsprays are solutions of polymer in ethanol/water. 0-5% is a whole mix of polymers, the rest is ethanol/water with water being anything from 0-45% of it.

Antibodies delivered in a spray format can subsequently be used for laundry, personal care (deodorants, antiperspirants, perfumes, hair products) or hygiene applications (hard surface cleaning).

### 3.5. The delivery of antibodies in a gel stick or roll-on format

Stick applicators or roll-ons (similar to those used in deodorants) could be used to apply an antibody or antigen and would also constitute a low water environment.

### 3.6. The delivery of antibodies in an absorbent pad.

Actives can either be delivered from or retained within an absorbent pad. In that case, the binding occurs in the presence of low levels of water.

### 3.7. The delivery of antibodies in a dry-cleaning process

Antibodies can also be used in a dry cleaning process, e.g. in recognising specific stains.

Again, antibodies delivered in this manner can be used in Laundry, personal care (deodorants, antiperspirants, perfumes, hair products) or hygiene applications (hard surface cleaning). The term antibodies includes multiple specificities on a single protein molecule e.g. biheads / tripleheads.

The compositions of the invention can be used in a detergent composition which is specifically suited for washing environments with low water content, and this constitutes a second aspect of the invention. When formulating an antibody containing product, it is important to ensure that the other ingredients of the product are compatible with antibody activity. WO-A-98/07820 (P&G) discloses inter alia rinse treatment compositions containing antibodies directed at cellulase and standard softener actives such as DEQA. The said invention preferably contains no softener or low levels of softener active (e.g. HEQ). If HEQ is present, the rinse product contains Sodium tripolyphosphate (STP) to stabilise antibody activity.

However, the present inventors achieved much superior binding and specificity in rinse liquors (or tap water) by omitting typical softener compositions. They also achieved improved binding in the presence of softener compositions by adding salts, especially multivalent salts such as STP. It is also very surprising that the inventors have found antibodies to be active in rinse liquors (or tap water). Previously published descriptions of specific antibody binding are typically in physiological strength buffer (0.15M NaCl) often supplemented with 0.15% surfactant. In many ways this mimics the environment in which the antibodies bind in nature, namely in serum which is approximately 0.15M NaCl, pH 7, and where serum albumin may be thought to act in an analogous way to a surfactant in that it reduces the opportunity for non-specific binding reactions.

To that extent, the composition comprises one or more benefit agents and optionally other conventional detergent ingredients. The invention in its second aspect provides a rinse composition which comprises from 0.1 - 50 % by weight, based on the total composition, of one or more surfactants. This surfactant system may in turn comprise 0 - 95 % by weight of one or more anionic surfactants and 5 - 100 % by weight of one or more nonionic surfactants. The surfactant system may additionally contain amphoteric or zwitterionic detergent compounds, but this in not normally desired owing to their relatively high cost. It was found to be advantageous to also include cationic surfactants into the composition. Examples of suitable cationic surfactants are given in WO-A-97/03160 and WO-A-98/17767 (Procter&Gamble).

In general, the nonionic and anionic surfactants of the surfactant system may be chosen from the surfactants described "Surface Active Agents" Vol. 1, by Schwartz & Perry, Interscience 1949, Vol. 2 by Schwartz, Perry & Berch, Interscience 1958, in the current edition of "McCutcheon's Emulsifiers and Detergents" published by Manufacturing Confectioners Company or in "Tenside-Taschenbuch", H. Stache, 2nd Edn., Carl Hauser Verlag, 1981.

Suitable nonionic detergent compounds which may be used include, in particular, the reaction products of compounds having a hydrophobic group and a reactive hydrogen atom, for example, aliphatic alcohols, acids, amides or alkyl phenols with alkylene oxides, especially ethylene oxide either alone or with propylene oxide. Specific nonionic detergent compounds are $C_6$-$C_{22}$ alkyl phenol-ethylene oxide condensates, generally 5 to 25 EO, i.e. 5 to 25 units of ethylene oxide per molecule, and the condensation products of aliphatic $C_8$-$C_{18}$ primary or secondary linear or branched alcohols with ethylene oxide, generally 5 to 40 EO.

Suitable anionic detergent compounds which may be used are usually water-soluble alkali metal salts of organic sulphates and sulphonates having alkyl radicals containing from about 8 to about 22 carbon atoms, the term alkyl being used to include the alkyl portion of higher acyl radicals. Examples of suitable synthetic anionic detergent compounds are sodium and potassium alkyl sulphates, especially those obtained by sulphating higher $C_8$-$C_{18}$ alcohols, produced for example from tallow or coconut oil, sodium and potassium alkyl $C_9$-$C_{20}$ benzene sulphonates, particularly sodium linear secondary alkyl $C_{10}$-$C_{15}$ benzene sulphonates; and sodium alkyl glyceryl ether sulphates, especially those ethers of the higher alcohols derived from tallow or coconut oil and synthetic alcohols derived from petroleum. The preferred anionic detergent compounds are sodium

$C_{11}$-$C_{15}$ alkyl benzene sulphonates and sodium $C_{12}$-$C_{18}$ alkyl sulphates. Also applicable are surfactants such as those described in EP-A-328 177 (Unilever), which show resistance to salting-out, the alkyl polyglycoside surfactants described in EP-A-070 074, and alkyl monoglycosides.

Preferred surfactant systems are mixtures of anionic with nonionic detergent active materials, in particular the groups and examples of anionic and nonionic surfactants pointed out in EP-A-346 995 (Unilever). Especially preferred is surfactant system which is a mixture of an alkali metal salt of a $C_{16}$-$C_{18}$ primary alcohol sulphate together with a $C_{12}$-$C_{15}$ primary alcohol 3-7 EO ethoxylate.

The nonionic detergent is preferably present in amounts greater than 10%, e.g. 25-90% by weight of the surfactant system. Anionic surfactants can be present for example in amounts in the range from about 5% to about 40% by weight of the surfactant system.

The rinsing detergent composition may take any suitable physical form, such as a powder, a tablet, an aqueous or non aqueous liquid, a paste or a gel. The complex of benefit agent and reagent having a high affinity according to the invention will generally be used as a dilution in water of about 0.05 to 2%.

The rinse composition in accordance with the invention comprising the complex of the reagent having a high affinity for the fabric and the benefit aid can have any suitable form, i.e. the form of a granular composition, a liquid or a slurry of the enzyme, or with carrier material (e.g. as in EP-A-258 068 and the Savinase (TM) and Lipolase (TM) products of Novo Nordisk). A good way of adding the complex to a liquid rinse product is in the form of a slurry containing from 0.005 to 50 % by weight of the complex in an ethoxylated alcohol nonionic surfactant.

The compositions of the invention comprise about 0.001 to 10 mg, preferably from 0.01 to 10 mg of antibody per liter of the wash liquor in use. A concentrated composition before use will comprise about 1 to 1000 mg/l, preferably from 10 mg to 100 mg per liter of the detergent product.

The invention will now be further illustrated in the following, non-limiting Examples.

## Example 1

Monoclonal antibody binding to antigen in low concentrations of water

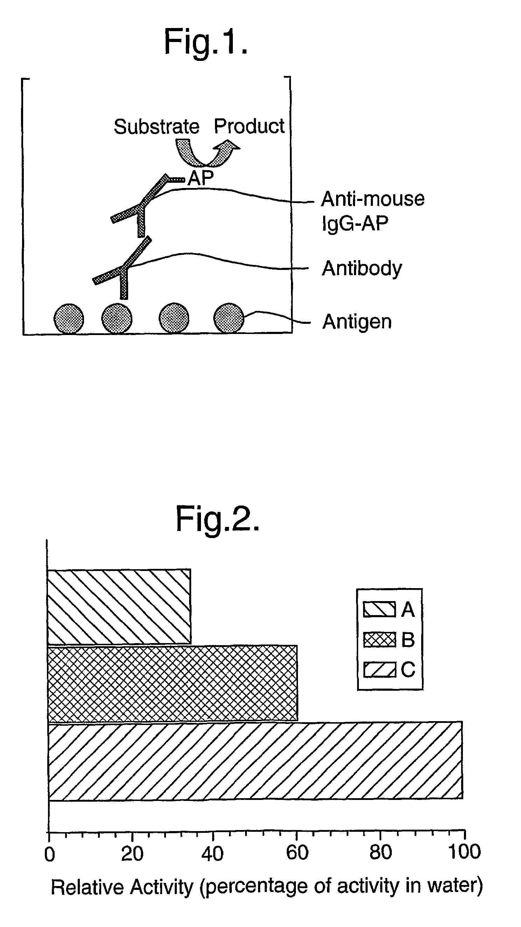

An ELISA plate was coated with 100 µl per well of human chorionic gonadotrophin (hCG, Sigma Chemical Co) diluted to 1000 mIU/ml in phosphate buffered saline (PBS) containing 0.1 % (v/v) Tween 20 and 0.02 % sodium azide (PBSTA). After an overnight incubation at 4°C the wells were washed 3 times with 200 µl per well PBSTA. The plate was blocked by incubating with 100 µl per well PBSTA containing 10 mg/ml bovine serum albumin (BSA) for 30 min at room temperature. The wells were then washed again 3 times with 200 µl per well PBSTA and the plate was incubated with various serial dilutions of a monoclonal antibody specific for hCG (MAb 3299) diluted in various concentrations of glycerol diluted in PBSTA. MAb 3299 was generated using standard monoclonal antibody techniques, however, monoclonal antibodies recognising hCG can be obtained from commercial suppliers. Following a 1 hour incubation at room temperature the plate was washed 3 times with PBSTA and then incubated with anti-mouse IgG-alkaline phosphatase conjugate (1:1000 dilution in PBSTA) for 1 hour at room temperature. Another 3 washes were carried out and the plates were incubated with substrate solution (1 tablet of Sigma Phosphatase 104 in 5 ml 1 M Diethanolamine containing 1 mM $MgCl_2$ at pH 8.5) for 30 min at room temperature. A schematic showing the stages of the assay are depicted in figure 1. The optical density of the product was measured at 405 nm (see figure 2; 10 µg/ml MAb 3299 made up in A, 100% glycerol; B, 70% glycerol; C, 0% glycerol).

## Example 2

Antigen binding to monoclonal antibody in low concentrations of water

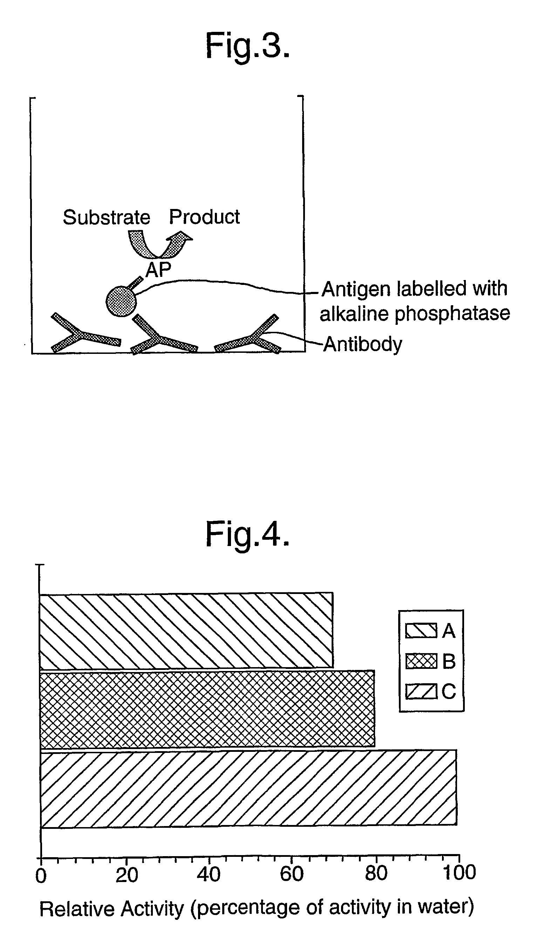

An ELISA plate was coated with a monoclonal antibody (MAb 3299, specific for hCG) at 100 µl per well diluted to 10 µg/ml in PBSTA. Following an overnight incubation at 4°C, the wells were washed 3 times with 200 µl per well PBSTA. The plate was blocked with 100 µl PBSTA containing 10 mg/ml BSA for 30 min at room temperature and then washed 3 times with PBSTA. The plate was then incubated for 1 hour at room temperature with hCG conjugated to alkaline phosphatase (serially diluted in various concentrations of glycerol made up in PBSTA). The plate was washed again and incubated with substrate solution (1 tablet of Sigma Phosphatase 104 in 5 ml 1 M Diethanolamine containing 1 mM $MgCl_2$ at pH 8.5) for 30 min at room temperature. A schematic showing the stages of the assay are depicted in figure 3. The optical density of the product was measured at 405 nm (see figure 4).

## Example 3

Antibody binding to antigen in low volumes of water using a spray applicator

Preparation of an antigen surface

Petri dishes of diameter 5 cm (approx 20 $cm^2$ surface area) were each incubated with 5 ml hCG (Sigma) diluted to 1000 mIU/ml for 1 hour at room temperature. The petri dishes were then washed 3 times with 5 ml PBSTA and blocked by incubating with 5 ml PBSTA containing 10 mg/ml BSA for 30 min at room temperature. Following a further 3 washes with PBSTA the dishes were tapped dry. Control petri dishes were prepared by following the same procedure as described for hCG application but by omitting the hCG in the dilutions.

Binding antibody to an antigen surface using a spray applicator

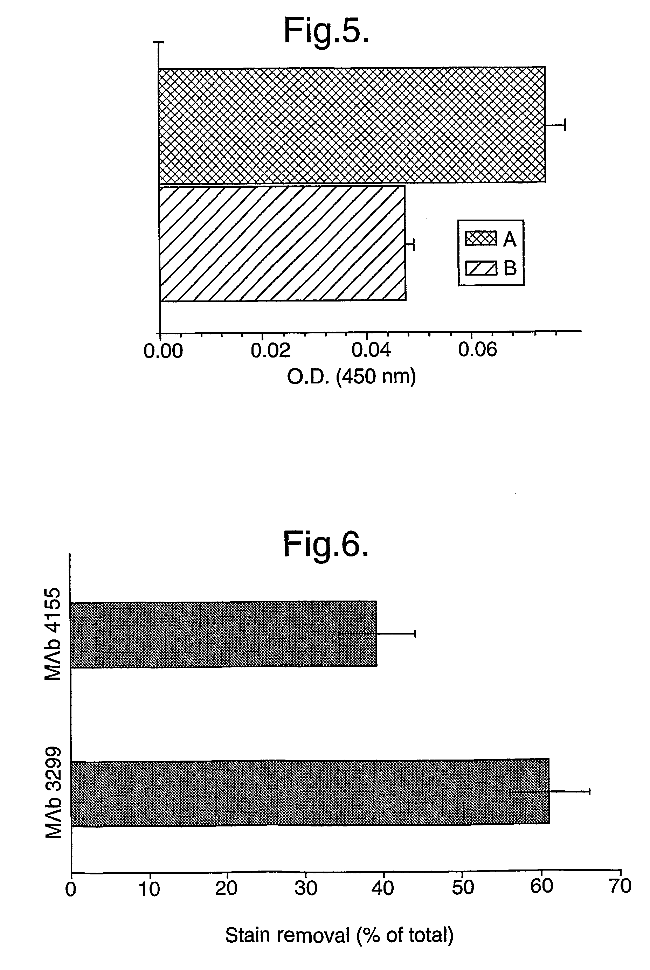

Each petri dish was sprayed using a spray applicator (available from garden centres for spraying plants) with a solution containing monoclonal antibody 3468 recognising hCG diluted to 10 μg/ml in PBSTA. The spray delivered between 48 - 65 μl (mean ± S.D. = 58 ± 8 μl) of reagent as determined by immediately weighing the petri dishes before and after antibody application. The antibody solution was allowed to dry overnight at 4°C and the dishes were then washed once more with PBSTA. Next, the dishes were incubated with anti-mouse IgG-alkaline phosphatase conjugate (Sigma, 1:1000 dilution in PBSTA) for 1 hour at room temperature. Following 3 washes with PBSTA the dishes were incubated with substrate solution (1 Sigma phopsphatase 104 tablet in 5 ml 1 M Diethanolamine containing 1 mM $MgCl_2$ at pH 8.5). After incubation for 210 minutes a 100 μl aliquot was taken from each dish and placed in an ELISA plate and the optical density at 410 nm was determined. Figure 5 shows the mean ± S.D. (n = 3) for the antigen coated dishes (A) and the control dishes (B).

Dry bind stain removal

**1.1 Preparation of antibody coated latex.**

A passive adsorption method was used to create antibody-coated latex. 100 μl of Duke Blue or Duke Green Scientific latex was mixed with 900 μl of borate buffer (0.01 M borate, 0.02 % thimerosol, pH 8.5). After 10 min centrifugation at 13,000 rpm the pellet was resuspended in 1 ml borate buffer. The solution was centrifuged again for 10 min at 13,000 rpm and the pellet was resuspended in 1 ml MAb 3299 or MAb 4155 diluted to 50 μg/ml in borate buffer. After a 2 hour incubation at room temperature with agitation on an end-over-end mixer, 200 μl of bovine serum albumin (BSA) diluted to 10 mg/ml in borate buffer was added. After a further 30 min incubation with agitation on an end-over-end mixer followed centrifugation for 10 min at 13000 rpm. The pellet was resuspended in 1 ml borate buffer then centrifuged again for 10 min at 13000 rpm. After a final resuspension in 1 ml borate buffer the antibody coated latex was stored at 4°C.

**1.2 Preparation of blue latex stained cloth.**

Swatches of white cotton were submersed in Duke Blue Scientific latex (10% solids of 400 nm) coated with human chorionic gonadotrophin (hCG, Sigma Chemical Co). After a two hour incubation at 37°C the swatches were removed and laid to dry at 37°C overnight.

**1.3 Stain removal in glycerol**

Two 1x1" swatches of stained cotton were rinsed separately in 50 ml glycerol. After 30 min of agitation 1 rinsed swatch was placed in 50 ml of glycerol containing 200 μl Duke Green Scientific latex (1% solids of 412 nm) coated with monoclonal antibody specific for hCG (Mab 3299). The second swatch was placed in 50 ml of glycerol containing 200 μl Duke Green Scientific latex (1% solids of 412 nm) coated with monoclonal antibody specific for oesterone 3 glucuronide (E3G) (Mab 4155). After a 1 hour incubation at room temperature with agitation the swatches were removed. The remaining glycerol was analysed using flow cytometry to detect and quantify the presence of blue latex (see figure 1). The total amount of stain removed is the amount of blue latex present in the 50 ml latex/glycerol/MAb 3299 solution plus the amount of blue latex present in the 50 ml latex/glycerol/MAb 4155. To compare the stain removal strength of each antibody the values are expressed as a percentage of the total amount of stain removed. The results are shown in Fig.6.

**Claims**

1. Process for binding an antigen to a molecule having a high binding affinity to said antigen, **characterized in that** the binding is carried out in a medium having a water content of less than 25% by weight of the total composition.

2. Process according to claim 1, wherein the medium has a water content of less than 15%, preferably less that 10% by weight of the total composition.

3. Process according to claim 1, wherein the medium has a water content of less than 5% by weight of the total composition.

4. Process according to any one of the preceding claims, wherein the reagent having a high binding affinity for the fabric is a protein or a peptide.

5. Process according to any one of the preceding claims, wherein the reagent having a high binding affinity is an antibody, an antibody fragment, or a derivative thereof.

6. Process according to any one of the preceding claims, wherein the reagent having a high binding affinity has a chemical equilibrium constant $K_d$ for the fabric of less than $10^{-4}$ M, preferably less than $10^{-6}$ M.

7. Process according to claim 6, wherein the chemical equilibrium constant $K_d$ is less than $10^{-7}$ M.

8. Process according to any one of the preceding claims, wherein the fabric is cotton, polyester, or polyester / cotton, or wool, or silk.

9. Process according to any one of the preceding claims, wherein the reagent having a high binding affinity is directed at a specific part of the fabric.

10. Process according to any one of the preceding claims, using micro-particles sensitised with antibody, and configured such that the micro-particles are loaded with the benefit agent.

11. Process according to any one of the preceding claims, whereby the reagent having a high binding affinity for the fabric is a multi-specific antibody or antibody or an analogous structure arranged so that at least one specificity is directed to the fabric and the others are directed to one or more benefit agents.

12. Process according to claim 10, wherein the reagent has one specificity directed to the fabric and one to the benefit agent.

**Patentansprüche**

1. Verfahren zum Binden eines Antigens an ein Molekül mit einer hohen Bindungsaffinität für das Antigen, **dadurch gekennzeichnet, dass** das Binden in einem Medium mit einem Wassergehalt von weniger als 25 Gewichtsprozent der Gesamtzusammensetzung ausgeführt wird.

2. Verfahren nach Anspruch 1, wobei das Medium einen Wassergehalt von weniger als 15%, vorzugsweise weniger als 10 Gewichtsprozent, der Gesamtzusammensetzung aufweist.

3. Verfahren nach Anspruch 1, wobei das Medium einen Wassergehalt von weniger als 5 Gewichtsprozent der Gesamtzusammensetzung aufweist.

4. Verfahren nach einem der vorangehenden Ansprüche, wobei das Reagens mit einer hohen Bindungsaffinität für das Textil ein Protein oder ein Peptid ist.

5. Verfahren nach einem der vorangehenden Ansprüche, wobei das Reagens mit einer hohen Bindungsaffinität ein Antikörper, ein Antikörperfragment oder ein Derivat davon ist.

6. Verfahren nach einem der vorangehenden Ansprüche, wobei das Reagens mit einer hohen Bindungsaffinität eine chemische Gleichgewichtskonstante $K_d$ für das Textil von weniger als $10^{-4}$ M, vorzugsweise weniger als $10^{-6}$ M, aufweist.

7. Verfahren nach Anspruch 6, wobei die chemische Gleichgewichtskonstante $K_d$ weniger als $10^{-7}$ M ist.

8. Verfahren nach einem der vorangehenden Ansprüche, wobei das Textil Baumwolle, Polyester oder Polyester/Baumwolle oder Wolle oder Seide ist.

9. Verfahren nach einem der vorangehenden Ansprüche, wobei das Reagens mit einer hohen Bindungsaffinität auf einen bestimmten Teil des Textils gerichtet ist.

10. Verfahren nach einem der vorangehenden Ansprüche unter Verwendung von Mikroteilchen, die mit Antikörper sensibilisiert wurden und derart konfiguriert, dass die Mikroteilchen mit dem Vorteil verleihenden Mittel beladen werden.

11. Verfahren nach einem der vorangehenden Ansprüche, wobei das Reagens mit einer hohen Bindungsaffinität für das Textil ein multispezifischer Antikörper oder Antikörper oder eine analoge Struktur darstellt, die derart angeordnet ist, dass mindestens eine Spezifität auf das Textil gerichtet ist und die anderen auf ein oder mehrere Vorteil verleihende Mittel gerichtet sind.

12. Verfahren nach Anspruch 11, wobei das Reagens eine Spezifität, die auf das Textil und eine, die auf das Vorteil verleihende Mittel gerichtet ist, aufweist.

**Revendications**

1. Procédé servant à fixer un antigène à une molécule présentant une affinité élevée de fixation audit antigène, **caractérisé en ce que** la fixation est exécutée dans un milieu dont la teneur en eau est inférieure à 25 % en poids de la totalité de la composition.

2. Procédé selon la revendication 1, dans lequel le milieu a une teneur en eau inférieure à 15%, de préférence inférieure à 10% en poids de la totalité de la composition.

3. Procédé selon la revendication 1, dans lequel le milieu a une teneur en eau inférieure à 5% en poids de la totalité de la composition.

4. Procédé selon l'une quelconque des revendications précédentes, dans lequel le réactif présentant une affinité élevée de fixation vis à vis du tissu est une protéine ou un peptide.

**5.** Procédé selon l'une quelconque des revendications précédentes, dans lequel le réactif présentant une affinité élevée de fixation est un anticorps, un fragment d'anticorps, ou un dérivé de ceux-ci.

**6.** Procédé selon l'une quelconque des revendications précédentes, dans lequel le réactif présentant une affinité élevée de fixation a une constante d'équilibre chimique $K_d$ vis à vis du tissu inférieure à $10^{-4}$ M, de préférence inférieure à $10^{-6}$ M.

**7.** Procédé selon la revendication 6, dans lequel la constante d'équilibre chimique $K_d$ est inférieure à $10^{-7}$ M.

**8.** Procédé selon l'une quelconque des revendications précédentes, dans lequel le tissu est du coton, du polyester, ou du polyester/coton, ou de la laine, ou de la soie.

**9.** Procédé selon l'une quelconque des revendications précédentes, dans lequel le réactif présentant une affinité élevée de fixation est dirigé vers une partie spécifique du tissu.

**10.** Procédé selon l'une quelconque des revendications précédentes, utilisant des micro-particules sensibilisées à l'aide d'un anticorps, et configurées tel que les micro-particules sont chargées avec l'agent d'amélioration.

**11.** Procédé selon l'une quelconque des revendications précédentes, par lequel le réactif présentant une affinité élevée de fixation vis à vis du tissu est un anticorps multispécifique ou un anticorps ou une structure analogue disposée de sorte qu'au moins une spécificité soit envers le tissu et les autres soient envers un ou plusieurs agents d'amélioration.

**12.** Procédé selon la revendication 11, dans lequel le réactif a une spécificité envers le tissu et une spécificité envers l'agent d'amélioration.

# Fig.1.

# Fig.2.

Relative Activity (percentage of activity in water)

# Fig.3.

Substrate   Product

AP

Antigen labelled with alkaline phosphatase

Antibody

# Fig.4.

A
B
C

0   20   40   60   80   100

Relative Activity (percentage of activity in water)

# Fig.5.

O.D. (450 nm)

# Fig.6.

Stain removal (% of total)